(19)

Europäisches
Patentamt
European
Patent Office
Office européen
des brevets

(11) **EP 2 348 076 A1**

(12) **EUROPEAN PATENT APPLICATION**
published in accordance with Art. 153(4) EPC

(43) Date of publication:
**27.07.2011 Bulletin 2011/30**

(21) Application number: **09809907.0**

(22) Date of filing: **25.08.2009**

(51) Int Cl.:
*C09C 3/06* (2006.01)     *A61K 8/19* (2006.01)
*A61K 8/25* (2006.01)     *A61K 8/26* (2006.01)
*A61Q 1/02* (2006.01)     *A61Q 3/02* (2006.01)
*C09C 1/00* (2006.01)     *C09D 5/29* (2006.01)
*C09D 5/33* (2006.01)     *C09D 7/12* (2006.01)
*C09D 11/00* (2006.01)     *C09D 201/00* (2006.01)

(86) International application number:
**PCT/JP2009/064802**

(87) International publication number:
**WO 2010/024256 (04.03.2010 Gazette 2010/09)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(30) Priority: **25.08.2008 JP 2008215889**

(71) Applicant: **Nippon Sheet Glass Company Limited Tokyo 108-6321 (JP)**

(72) Inventors:
• **Kitamura Takeaki**
  **Tokyo 108-6321 (JP)**
• **HIOKi Masahiro**
  **Tokyo 108-6321 (JP)**

(74) Representative: **Matthews, Derek Peter**
  **Dehns**
  **St Bride's House**
  **10 Salisbury Square**
  **GB-London EC4Y 8JD (GB)**

(54) **SILVER-BASED PHOTOLUMINESCENT PIGMENT AND A COSMETIC COMPOSITION, PAINT COMPOSITION, ADIABATIC PAINT COMPOSITION, INK COMPOSITION, AND RESIN COMPOSITION CONTAINING SAME**

(57)     A bright pigment with improved brightness is provided. A bright pigment 1 of the present invention includes a scaly inorganic base 10 and a coating 20 containing a pure substance of silver or a silver alloy and covering the inorganic base 10. The inorganic base 10 has an average thickness of 0.2 μm to 7.0 μm. The content of the pure substance of silver or the silver alloy in the bright pigment 1 satisfies the formulas 1) and 2):
    1)

$$y \geq -9 \operatorname{Ln}(x) + 19$$

2)

$$y \leq -22 \operatorname{Ln}(x) + 52$$

where x is the average thickness (μm) of the inorganic base and y is the content (mass%) of the pure substance of silver or the silver alloy.

FIG. 1

**Description**

Technical Field

The present invention relates to a silver-based bright pigment, and further to cosmetic, paint, thermal insulation paint, ink and resin compositions containing the silver-based bright pigment.

Background Art

Conventionally known bright pigments include scaly aluminum particles, graphite particles, scaly glass particles, silver-coated scaly glass particles, natural mica, silica, and alumina particles coated with a metal oxide, such as titanium dioxide or iron oxide.
Such bright pigments reflect light on their surface and have glitter properties and, therefore, are used as ingredients in paint, ink, resin and cosmetic compositions, among others. When forming a coating using a cosmetic composition containing such bright pigments, the coating gives, in combination with the background color, a varied, unique appearance with excellent aesthetic properties.
As bright pigments having a metallic luster, those specified in (1) and (2) below are particularly known. As the coating metal described below, gold, silver, copper, nickel, chrome, molybdenum, aluminum, tin, magnesium, or a mixture thereof is used. Since these bright pigments have a metallic luster, they can impart strong brightness to objects to which the pigments are applied and provide the objects with an excellent outer appearance in terms of design.

(1) powder obtained by pulverizing an aluminum powder or a thin-film resin covered with metal
(2) a scaly base, such as glass, natural mica, synthetic mica, silica and alumina, covered with metal

Specifically for example, the following bright pigments are proposed (e.g., Patent Documents 1 to 5).
Patent Document 1 discloses a bright pigment in which scaly glass is coated with metal on its surface by sputtering. The metal coating is made of Au, Ag, Cu, Al or an alloy thereof and has a thickness of 50 to 200 Å (5 to 20 nm). The scaly glass has an average particle size of 10 to 300 $\mu$m and an average thickness of 1 to 20 $\mu$m.
Patent Document 2 discloses a bright pigment in which scaly glass is coated with metal on its surface and a makeup cosmetic composition containing the bright pigment. The metal coating is made of Ag, Ni, Cr, Mo, Al, Au, Cu, Sn, Mg or a mixture thereof. The amount of the metal with which the scaly glass is coated is 0.1 to 50 wt%, preferably 1 to 20 wt%, and more preferably 2 to 8 wt%. The scaly glass has a thickness of 0.1 to 25 $\mu$m, preferably 0.5 to 5 $\mu$m, and an average particle size of 1 to 500 $\mu$m, preferably 25 $\mu$m to 150 $\mu$m.
Patent Document 3 discloses a bright pigment in which flaky glass is coated with metal and a cosmetic composition containing the bright pigment. The metal coating is made ofAg, Au, Ni or Hastelloy (registered trademark). The flaky glass has an average thickness of 0.3 to 8 $\mu$m and an average particle size of 5 to 5000 $\mu$m.
Patent Document 4 discloses a silver metallic pigment in which a granular base of flaky glass or mica is coated with a silver alloy by physical vapor deposition. The silver alloy contains 0.5 to 10 wt% of one or more metals selected from the group consisting of Al, Cr, Ni, Ti and Mg.
Moreover, Patent Document 5 discloses a pigment in which natural mica is coated with noble metal on its surface by chemical plating and a cosmetic composition containing the pigment. The noble metal is at least one selected from the group consisting of Au, Pt, Pd and Ag, and the amount of the noble metal with which the natural mica is coated is 5 to 70 wt%. The natural mica has an average particle size of 20 to 300 $\mu$m.

Prior Art

Patent Document

Patent Document 1: JP H9-188830 A
Patent Document 2: JP 2001-089324 A
Patent Document 3: JP 2002-138010 A
Patent Document 4: JP H10-158540 A
Patent Document 5: JP H6-76292 B

Disclosure of Invention

Problem to be Solved by the Invention

However, these conventional bright pigments have inadequate brightness, and therefore a further improvement in the brightness is required. Therefore, the present invention provides a bright pigment with improved brightness as well as cosmetic, paint, thermal insulation paint, ink, and resin compositions containing the bright pigment.

Means for Solving Problem

The bright pigment of the present invention is a bright pigment including: a scaly inorganic base; and a coating containing a pure substance of silver or a silver alloy and covering the inorganic base. The inorganic base has an average thickness of 0.2 $\mu$m to 7.0 $\mu$m. The content of the pure substance of silver or the silver alloy in the bright pigment satisfies the formulas 1) and 2):

   1)

$$y \geq -9 \, \mathrm{Ln}\,(x) + 19$$

   2)

$$y \leq -22 \, \mathrm{Ln}\,(x) + 52$$

where x is the average thickness ($\mu$m) of the inorganic base and y is the content (mass%) of the pure substance of silver or the silver alloy.

Further, viewed from another aspect, the present invention is a cosmetic composition containing the bright pigment of the present invention.

Further, viewed from still another aspect, the present invention is a paint composition containing the bright pigment of the present invention.

Further, viewed from yet another aspect, the present invention is a thermal insulation paint composition containing the bright pigment of the present invention.

Further, viewed from yet another aspect, the present invention is an ink composition containing the bright pigment of the present invention.

Further, viewed from yet another aspect, the present invention is a resin composition containing the bright pigment of the present invention.

Effects of the Invention

According to the present invention, it is possible to provide a bright pigment with improved brightness as well as cosmetic, paint, thermal insulation paint, ink and resin compositions containing the bright pigment.

Brief Description of Drawings

   [FIG. 1] FIG. 1 is a schematic cross-sectional view of one example of the bright pigment of the present invention.
   [FIG. 2] FIG. 2 is a schematic diagram for explaining a luminance measurement method employed in Examples.
   [FIG. 3] FIG. 3 is a graph showing the relationship between an inorganic base average thickness and a silver content of bright pigments of Examples 1 to 12 and Comparative Examples 1 to 8.

Description of the Invention

The present invention is based upon the following findings. As for a bright pigment in which a scaly inorganic base is coated with a pure substance of silver or a silver alloy, if the inorganic base has an average thickness of 0.2 $\mu$m

to 7.0 μm and the content of the pure substance of silver or the silver alloy (hereinafter also referred to as the "silver or silver alloy content") in the bright pigment is within the ranges of the aforementioned formulas 1) and 2) defined by the average thickness of the inorganic base, it is possible to provide a bright pigment that exhibits high brightness.

Therefore, the bright pigment of the present invention exhibits high luminance design. Particularly, when the bright pigment is used together with an organic colorant, it is possible to provide cosmetic, paint, thermal insulation paint, resin and ink compositions, an artificial marble molded product and coated paper that have a glittering vivid color and a refined design. Cosmetic, paint, thermal insulation paint, resin and ink compositions, an artificial marble molded product and coated paper that contain the bright pigment of the present invention and an organic colorant provide a refined design with a glittering vivid color, which cannot be achieved from an organic colorant alone.

Further, according to the bright pigment of the present invention, the inorganic base constituting the bright pigment has an average thickness of 0.2 μm to 7.0 μm. Thus, it is possible to provide, for example, a cosmetic composition having both high luminance and high chroma. Furthermore, when the inorganic base has an average thickness of 0.2 μm or more and less than 1.0 μm, it is possible to provide, for example, a cosmetic composition having both higher luminance and higher chroma.

(Embodiment 1)

In Embodiment 1, one example of the bright pigment according to the present invention will be described.

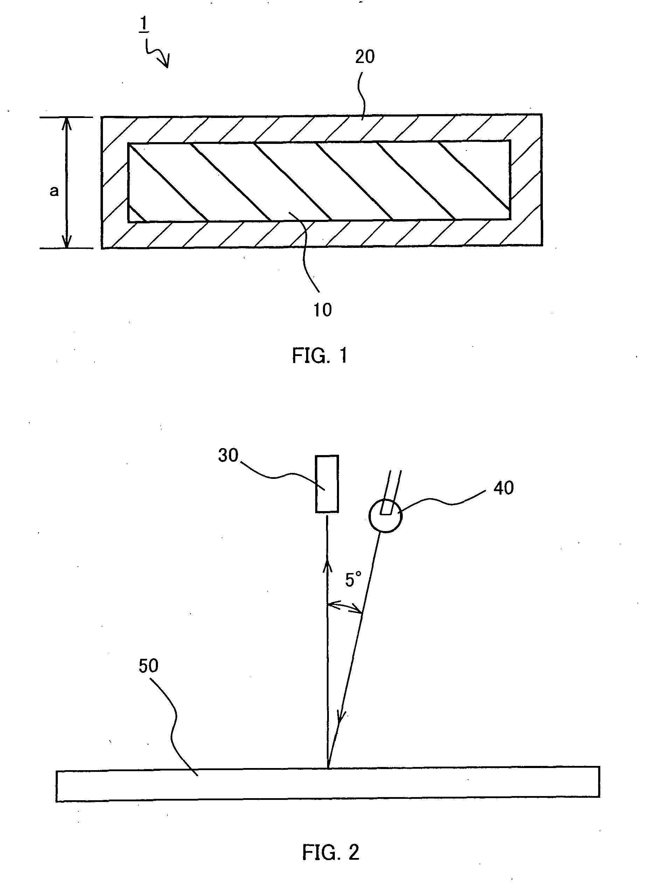

The bright pigment of the present invention includes a scaly inorganic base and a coating containing a pure substance of silver or a silver alloy (hereinafter, referred also to as the "silver-containing coating") and covering the inorganic base. FIG. 1 is a schematic cross-sectional view of one example of the bright pigment of the present invention. As shown in FIG. 1, a bright pigment 1 includes a scaly inorganic base 10 and a silver-containing coating 20 covering the inorganic base 10.

(Inorganic Base)

The inorganic base includes at least one selected from the group consisting of glass (specific gravity: 2.4), natural mica (specific gravity: 2.7), synthetic mica (specific gravity: 2.7), silica (specific gravity: 2.2) and alumina (specific gravity: 2.4). Preferably, the inorganic base is substantially composed of at least one material selected from the group consisting of glass, natural mica, synthetic mica, silica and alumina. Among these materials, glass is preferable because a smooth surface can be easily obtained. The composition of glass is not particularly limited as long as a known glass composition from which scaly glass can be formed is used. Examples of glass compositions having favorable scaly glass formability include glass compositions C and E used for glass fibers. Japanese Industrial Standard (JIS) R3410 specifies each of C glass and E glass. Generally, C glass refers to glass having an alkaline oxide ($Na_2O + K_2O$) content of about 5 to 20% and is also called alkali-containing glass. E glass refers to glass compositions having an alkali ($Na_2O$, $K_2O$) content of 2.0% or less and is also called non-alkali glass.

For the inorganic base, a scaly glass base produced by blowing is preferable because it has a smooth surface and reflects light favorably. In blowing, a raw cullet is melted. The molten glass is discharged continuously from a circular slit while gas, such as air, is blown into the glass through blow nozzles formed in the interior of the circular slit. As a result, the molten glass is stretched and expanded to have a balloon shape. The balloon shaped glass is pulverized to obtain a scaly glass base. Examples of commercially available scaly glass bases produced by blowing include MICROGLAS (registered trademark) GLASFLAKE (registered trademark) series (RCF-160, REF-160, RCF-015, REF-015) manufactured by Nippon Sheet Glass Co., Ltd.

The inorganic base has an average thickness of 0.2 μm to 7.0 μm. The inorganic base has an average thickness of preferably 0.2 μm or more and less than 1.3 μm, more preferably 0.2 μm or more and less than 1.0 μm, still more preferably 0.2 μm or more and 0.8 μm or less, and still even more preferably 0.2 μm or more and 0.7 μm or less. This is because, for example, asperities on the coating film surface can be retarded, so that favorable feeling in use can be acheived. Furthermore, the number of particles of the bright pigment in the coating film can be increased, so that the luminance and color (chroma) of the coating film can be improved. When the bright pigment is used in a cosmetic composition, the inorganic base has an average thickness of preferably 0.2 μm to 7.0 μm, more preferably 0.2 μm to 3.0 μm, still more preferably 0.2 μm or more and less than 1.0 μm, still even more preferably 0.2 μm or more and 0.8 μm or less, and yet still even more preferably 0.2 μm or more and 0.7 μm or less. This is because, on the surface of keratin constituting skin, nails, lips, etc., the cosmetics become favorably flexible and their feeling of use also becomes favorable. Further, when the bright pigment is used in an ink composition, the inorganic base has an average thickness of preferably 0.2 μm to 3.0 μm, more preferably 0.2 μm or more and less than 1.0 μm, still more preferably 0.2 μm or more and 0.8 μm or less, and still even more preferably 0.2 μm or more and 0.7 μm or less. This is because the finished quality (smoothness) on the printing surface becomes favorable. Further, when the bright pigment is used in an ink composition or thermal insulation paint composition, the inorganic base has an average thickness of preferably 0.2 μm

to 3.0 μm, more preferably 0.2 μm or more and less than 1.0 μm, still more preferably 0.2 μm or more and 0.8 μm or less, and still even more preferably 0.2 μm or more and 0.7 μm or less. This is because the reflectivity in the near-infrared region can be improved.

The average thickness of the inorganic base is determined as follows. Based on the cross-section of the bright pigment observed with a simple scanning electron microscope (SEM), the thickness of the inorganic base is measured and an average thickness is determined based on 30 grains of the bright pigment.

The average particle size of the inorganic base is not particularly limited and can be determined appropriately in response to how the bright pigment is used. Generally, however, the inorganic base has an average particle size of preferably 1 μm to 500 μm, and more preferably 10 μm to 200 μm. When the average particle size is within the range mentioned, it is possible to prevent shattering of the bright pigment in the course of preparation. Further, the principle plane of the bright pigment in a coating film, a resin composition or the like is to be oriented in substantially a constant direction. Thus, a sufficient amount of light is reflected from the bright pigment, so that adequate brightness can be obtained.

When the bright pigment is used in a cosmetic composition, the average particles size of the inorganic base can be determined appropriately in response to the type of the cosmetic, etc. The inorganic base has an average particle size of preferably 20 μm to 200 μm, and more preferably 20 μm to 150 μm because the glitter of the bright pigment becomes stronger and a cosmetic composition with a more glittering particle feeling can be achieved. Further, when the bright pigment is used in an ink composition, the inorganic base has an average particle size of preferably 1 μm to 40 μm, and more preferably 15 μm to 35 μm. This is because the suitability for printing, such as the ability to prevent plate fogging, becomes favorable. In this specification, plate fogging refers to a phenomenon in which ink on the plate cannot be adequately scraped off by a doctor blade during the printing, so that the ink is transferred to impression paper, thereby causing scumming of printed matters.

The average particle size of the inorganic base is measured with a laser diffraction type particle size meter, and it corresponds to a particle diameter at which the cumulative volume of particles reaches 50% in a particle size distribution (D50).

(Silver-containing Coating)

The silver-containing coating contains a pure substance of silver or a silver alloy. The silver-containing coating may be made substantially of a pure substance of silver or a silver alloy. In the present invention, the coating being made substantially of a pure substance of silver or a silver alloy means that the coating is made only of a pure substance of silver or a silver alloy except unavoidable impurities contained in the coating raw material and/or unavoidable impurities blended into the coating in the course of production. More specifically, the silver-containing coating has a pure substance of silver or silver alloy content of, for example, 99 atomic% or more, preferably 99.5 atomic% or more, and more preferably approximately 100 atomic%.

The silver alloy contains, for example, silver and other noble metal. The content of other noble metal in the silver alloy is preferably 0.1 atomic% or more, and more preferably 0.1 to 2.0 atomic%. Examples of other noble metals include gold, palladium, platinum, copper, rhodium, iridium, ruthenium and osmium. Examples of the silver alloy include an alloy of silver and gold, an alloy of silver and palladium, an alloy of silver and platinum, an alloy of silver and copper, an alloy of silver, gold and palladium, an alloy of silver, platinum and palladium, an alloy of silver, copper and palladium, an alloy of silver, gold and copper, and an alloy of silver, gold and platinum.

The silver-containing coating has an average thickness of preferably 20 nm to 100 nm, and more preferably 25 nm to 90 nm. This is because an increase in the cost due to the formation of the silver-containing coating can be cut down while the amount of light to be reflected can be increased effectively, so that the brightness of the bright pigment can be improved. The average thickness of the silver-containing coating can be determined as follows. The thickness of the silver-containing coating is measured based on the cross-section of the bright pigment observed with a field emission scanning electron microscope (FE-SEM) and an average thickness is determined based on 10 grains of the bright pigment. For example, S-4800 (manufactured by Hitachi High-Technologies) can be used as the field emission scanning electron microscope.

The silver-containing coating can be formed by sputtering, chemical-vapor deposition, electroless (chemical) plating, etc. Among these methods, electroless plating is preferable because a uniform coating can be formed easily on the scaly inorganic base.

For example, the following compounds can be used as the metal material of a plating solution used in electroless plating. When forming a silver alloy coating, a mixture of the following compounds may be used.

(1) silver material silver nitrite
(2) gold material: sodium gold(I) sulfite, gold(III) chloride[tetrachloro-gold(III) tetrahydrate]
(3) palladium material: diamino-palladium nitrite, palladium(II) chloride, palladium(II) nitrate, tetraamine palladium(II) dichloride
(4) platinum material platinum(IV) chloride[hexachloro platinum(IV) hexahydrate], dinitro diamine platinum(II), tetraaminedichloro platinum(II)
(5) copper material: copper nitrate, copper chloride, copper sulfate, copper acetate It is desirable to avoid using a cyanide in terms of safety.

(Silver or Silver Alloy Content)

The silver or silver alloy content in the bright pigment needs to satisfy the following formulas 1) and 2), and preferably satisfies the following formulas 3) and 4). In terms of improving the luminance and chroma, it is more preferable that the content satisfies the following formulas 5) and 6).

1)

$$y \geq -9 \, \mathrm{Ln}\,(x) + 19$$

2)

$$y \leq -22 \, \mathrm{Ln}\,(x) + 52$$

3)

$$y \geq -9.0569 \, \mathrm{Ln}\,(x) + 19.481$$

4)

$$y \leq -21.734 \, \mathrm{Ln}\,(x) + 52.254$$

5)

$$y \geq -12 \, \mathrm{Ln}\,(x) + 29$$

6)

$$y \leq -17 \, \mathrm{Ln}\,(x) + 41$$

where x is the average thickness ($\mu$m) of the inorganic base and y is the content (mass%) of the pure substance of silver or the silver alloy.

When the inorganic base has an average thickness of 0.2 $\mu$m or more and less than 0.35 $\mu$m, the silver or silver alloy content is preferably 28.5 to 87 mass% of the bright pigment. When the inorganic base has an average thickness of 0.35 $\mu$m or more and less than 0.55 $\mu$m, the silver or silver alloy content is preferably 24 to 70 mass%, and more preferably 30 to 60 mass% of the bright pigment. When the inorganic base has an average thickness of 0.55 $\mu$m or more and less than 0.75 $\mu$m, the silver or silver alloy content is preferably 23 to 60 mass%, and more preferably 35 to 60 mass% of the bright pigment. When the inorganic base has an average thickness of 0.75 $\mu$m or more and less than 1.0 $\mu$m, the silver or silver alloy content is preferably 20 to 58 mass%, and more preferably 45 to 55 mass% of the bright pigment.

The silver or silver alloy content of the bright pigment can be measured by totally dissolving the bright pigment in aqua regia and measuring the silver or silver alloy content with an inductively coupled plasma mass spectrometer ("ICP"-MS).

For example, the bright pigment of the present invention can be added to, for example, cosmetic, ink, paint, thermal insulation paint, and resin compositions. In addition to these compositions, the bright pigment of the present invention can also be used as a material of water-borne paints, oil paints, genuine leather, synthetic leather, textile printing, buttons, lacquer ware, pigments for chinaware, etc.

In terms of achieving both high luminance and a vivid color on surfaces given a makeup, printing surfaces, painting surfaces, and a surface of resin molded products, the bright pigment of the present invention is preferably added to cosmetic, ink, paint, thermal insulation paint, and resin compositions together with an organic colorant. As the organic colorant, it is preferable to use organic colorants used in food products specified by the Japanese Food Sanitation Act and pharmaceuticals, quasi-medical products and cosmetics specified by the Japanese Pharmaceutical Affairs Act. Further, an organic synthetic colorant (tar colorant) can be used as the organic colorant.

Next, one method for producing the bright pigment of the present invention will be described by way of example.

The bright pigment of the present invention can be produced by coating a scaly inorganic base with a pure substance of silver or a silver alloy. As described above, sputtering, CVD, and electroless (chemical) plating, among others, can be employed in forming the silver-containing coating. Above all, electroless plating is preferable.

In electroless plating, the silver-containing coating can be formed as follows. A pretreated inorganic base is added to a plating solution containing a metal material and a pure substance of silver or a silver alloy is deposited on the surface of the inorganic base by an electroless plating reaction. The inorganic base can be pretreated with an aqueous solution of tin(II) chloride. It is possible to adjust the amount of a pure substance of silver or a silver alloy with which the inorganic base is coated (the silver or silver alloy content of the bright pigment) by controlling the concentration of tin (III) chloride in the aqueous solution used in the pretreatment, the amount of the inorganic base added to the plating solution and the concentration of the metal material in the plating solution. This allows the silver or silver alloy content to satisfy the aforementioned formulas 1) and 2), and a result, a bright pigment that exhibits high brightness can be provided.

Thus, viewed from another aspect, the present invention is a method for producing a bright pigment, including the step of coating a scaly inorganic base with a pure substance of silver or a silver alloy by electroless plating, where the inorganic base has an average thickness of 0.2 $\mu$m to 7.0 $\mu$m. In the coating step, the amount of the pure substance of silver or the silver alloy with which the inorganic base is coated is adjusted so that the content of the substance of silver or the silver alloy in the bright pigment satisfies the formulas 1) and 2):

1)

$$y \geq -9\,\mathrm{Ln}\,(x) + 19$$

2)

$$y \leq -22\,\mathrm{Ln}\,(x) + 52$$

where x is the average thickness ($\mu$m) of the inorganic base and y is the content (mass%) of the pure substance of silver or the silver alloy. The inorganic base has an average thickness of preferably 0.2 $\mu$m or more and less than 1.0 $\mu$m, more preferably 0.2 $\mu$m or more and 0.8 $\mu$m or less, and still more preferably 0.2 $\mu$m or more and 0.7 $\mu$m or less.

(Embodiment 2)

In Embodiment 2, one example of the cosmetic composition of the present invention will be described.

The cosmetic composition of the present invention contains the bright pigment of the present invention. The cosmetic composition may contain a medium as needed. Examples of the cosmetic composition include: nail cosmetics, facial cosmetics, makeup cosmetics and care cosmetics. Examples of nail cosmetics include nail polish, nail colors and nail coatings. Examples of makeup cosmetics include: eye and eyebrow cosmetics such as eye shadows, eyeliners, eyeliner pencils, mascara and eyebrow pencils; foundations; blushes; face colors; lipsticks; lip glosses; lip liners; and makeup cosmetics of a sedimentation type that are kept in a state where a lamé agent is sediment in water or a solvent but are adequately shaken to be used. Particularly, the cosmetic composition of the present invention is suited for: nail cosmetics such as nail polish, nail colors, and nail coatings; foundations such as makeup base and face powder; and makeup cosmetics such as eye shadow, eye liners, mascara, lipsticks and fancy powders. Cosmetics may be in any forms including powder, cake, pencil, stick, ointment, liquid, emulsion and cream forms.

The content of the bright pigment of the present invention in the cosmetic composition can be determined appropriately in response to the type of the cosmetic composition. In the case of nail cosmetics, the content of the bright pigment of the present invention is preferably 0.1 to 50 wt%, and more preferably 3 to 40 wt% of the total weight of the cosmetic composition in terms of achieving both high brightness and favorable applicability.

In the case of solid powder cosmetics, the content of the bright pigment of the present invention is preferably 5 to 80 wt%, and more preferably 10 to 60 wt% of the total weight of the cosmetic composition in terms of achieving both high brightness and favorable feeling in use. Examples of solid powder cosmetics include eye shadows obtained by drying a powder dry-filled by a press or wet-filled using a volatile solvent.

In the case of powder cosmetics, they are to be mixed with sebum present on the skin when used. Therefore, the content of the bright pigment of the present invention is preferably 70 wt% or more, and may be 100 wt% of the total weight of the cosmetic composition. Examples of powder cosmetics include eye shadows and face colors used as loose powders.

In the case of oily solid cosmetics, the content of the bright pigment of the present invention is preferably 1 to 60 wt%, and more preferably 3 to 50 wt% of the total weight of the cosmetic composition in terms of adequately ensuring the effect of the bright pigment of the present invention as a lamé agent and favorable formability. Examples of oily solid cosmetics include lipsticks and oily eye shadows.

In the case of emulsion makeup cosmetics obtained by emulsifying a water phase and an oil phase with an activator, the content of the bright pigment of the present invention is preferably 1 to 50 wt%, and more preferably 3 to 40 wt% of the total weight of the cosmetic composition in terms of adequately ensuring the effect of the bright pigment of the present invention as a lamé agent and high emulsion stability.

In the case of aqueous makeup cosmetics, the content of the bright pigment of the present invention is preferably 0.1 to 60 wt%, and more preferably 1 to 40 wt% of the total weight of the cosmetic composition in terms of adequately ensuring the effect of the bright pigment of the present invention as a lamé agent and favorable feeling in use. Examples of aqueous makeup cosmetics include aqueous mascara and aqueous gel containing a water-soluble resin, a watersoluble resin emulsion and a thickener.

The medium can be determined appropriately in response to the type of the cosmetic composition. Examples of the medium include: ketones that are in the form of liquid at ambient temperature, such as methyl ethyl ketone, methyl isobutyl ketone, diisobutyl ketone, isophorone, cyclohexanone and acetone; alcohols that are in the form of liquid at ambient temperature, such as ethanol, isopropanol, diacetone alcohol, 2-butoxyethanol and cyclohexanol; glycols that are in the form of liquid at ambient temperature, such as ethylene glycol, propylene glycol, pentylene glycol and glycerol; propylene glycol ethers that are in the form of liquid at ambient temperature, such as propylene glycol monomethyl ether, propylene glycol monomethyl ether acetate, and dipropylene glycol mono-n-butyl ether; short chain esters (having a total of 3 to 8 carbon atoms) such as ethyl acetate, methyl acetate, propyl acetate, n-butyl acetate and isopentyl acetate; alkanes that are in the form of liquid at ambient temperature, such as decane, heptane, dodecane and cyclohexane; and cyclic aromatic compounds that are in the form of liquid at ambient temperature, such as toluene and xylene. In particular, alcohols and short chain esters are preferable in terms of safety.

The cosmetic composition of the present invention may contain hyaluronic acid, collagen, other moisturizing ingredients, solid oil, liquid oil and/or powders as needed. When the cosmetic composition of the present invention contains hyaluronic acid and collagen, it is preferable that other moisturizing ingredients, solid oil, liquid oil or powders are contained in the cosmetic composition within the bounds of not compromising the effect of preventing the production of a precipitate called polyion complex due to a carboxyl group (minus charge) of the hyaluronic acid and an amino group (plus charge) of the collagen being bonded to each other.

Examples of moisturizing ingredients include polyethylene glycol, propylene glycol, glycerin, 1,3-butylene glycol, hexylene glycol, xylitol, sorbitol, maltitol, chondroitin sulfate, mucoitinsulfuric acid, caronic acid, atelocollagen, cholesteryl-12-hydroxy stearate, sodium lactate, bile salt, dl-pyrrolidone carboxylate, diglycerol (EO) PO adduct, chestnut rose extract, yarrow extract, and melilot extract. They may be used alone or in combination of two or more.

Examples of solid oils include: hydrocarbons such as polyethylene wax, ethylene propylene copolymer, solid paraffin wax, ceresin wax, microcrystalline wax, Fischer-Tropsch wax, and montan wax; waxes such as carnauba wax, candelilla wax, beeswax, Japan wax and spermaceti; fats and oils such as cocoa butter, palm oil and beef tallow; higher fatty acids such as stearic acid, lauric acid, myristic acid and behenic acid; higher alcohols such as cetyl alchohol, stearyl alcohol, lauryl alcohol and behenyl alcohol; hydrogenated oils such as hydrogenated coconut oil and hydrogenated castor oil; esters such as methyl stearate, cetyl palmitate, pentaerythritol rosinate and propylene glycol distearate; and silicone waxes such as stearyl-modified polysiloxane and behenyl-modified polysiloxane. These solid oils may be used alone or in a combination of two or more.

Irrespective of the origin such as animal oil, vegetable oil or synthetic oil, examples of liquid oils include hydrocarbons, fats and oils, hydrogenated oils, ester oils, aliphatic acids, higher alcohols, silicone oils, fluorine-based oils, lanolin derivatives, oil-based gelling agents, lipophilic surfactants and oil-soluble UV absorbers. Specifically, examples of liquid oil include: hydrocarbons such as liquid paraffin, squalane, vaseline, polyisobutylene and polybutene; fats and oils such as olive oil, castor oil, jojoba oil, mink oil and Macadamia nut oil; esters such as cetyl isooctanate, isopropyl myristate, isopropyl palmitate, octyldodecyl myristate, glyceryl trioctanate, diglyceryl diisostearate, diglyceryl triisostearate, glyceryl tribehenate, pentaerythritol rosinate, neopentyl glycol dioctanate, cholesterol fatty acid ester and di(cholesteryl-behenyl-octyldodecyl) N-lauroyl-L-glutamate; aliphatic acids such as isostearyl acid and oleic acid; higher alcohols such as oleyl alcohol and isostearic alcohol; silicones such as dimethylpolysiloxane oiligomer, dimethylpolysiloxane high polymer, methylphenyl polysiloxane, decamethyl cyclo pentasiloxane, octamethyl cyclo tetrasiloxane, polyether-modified polysiloxane, cross-linked organopolysiloxane, and fluorine-modified silicone; fluorine-based oils such as perfluoropolyether, perfluorodecane and perfluorooctane; lanoline derivatives such as lanoline acetate, lanoline fatty acid isopropyl and lanoline alcohol; oil-based gelling agents such as dextrin fatty acid ester, sucrose fatty acid ester, starch fatty acid ester, aluminum 12-hydroxystearate, and calcium stearate; and oil-soluble UV absorbers such as ethyl para-aminobenzoate, p-methoxycinnamic-2-ethylhexyl, 4-tert-butyl-4'-methoxy-dibenzoylmethane and oxybenzone. These liquid oils may be used alone or in a combination of two or more.

Examples of powders include inorganic powders, bright powders other than the bright pigment of the present invention, organic powders, composite powders and dye powders. These powders may be surface treated with, for example, a metallic oxide, a metallic hydroxide, a fluorine compound, silicone oil, metallic soap, wax, fats and oils or hydrocarbon. These powders may be used alone or in combination of two or more. Powders may have any shape including spherical, plate and needle shapes and their particle size may be smaller than or as large as the particle size of the bright pigment of the present invention. Further, they may have either a porous or nonporous structure.

Examples of inorganic powders include titanium oxide powder, black titanium oxide powder, iron blue powder, ultramarine blue powder, red oxide powder, yellow iron oxide powder, black iron oxide powder, zinc oxide powder, aluminum oxide powder, magnesium oxide powder, zirconium oxide powder, magnesium carbonate powder, calcium carbonate powder, chromium oxide powder, chromium hydroxide powder, carbon black powder, aluminum silicate powder, magnesium silicate powder, aluminum-magnesium silicate powder, mica powder, synthetic mica powder, synthetic sericite powder, sericite powder, talc powder, kaoline powder, silicic anhydride powder, silica bead powder, silicon carbide powder, barium sulfate powder, bentonite powder, smectite powder, and boron nitride powder.

Examples of bright powders include bismuth oxychloride powder, natural mica-titanium powder, natural mica powder coated with iron oxide, natural mica-titanium powder coated with iron oxide, natural mica-titanium powder treated with an organic colorant and aluminum powder.

Examples of organic powders include nylon powder, polymethyl methacrylate, acrylonitrile-methacryl copolymer powder, vinylidene chloride-methacrylic acid copolymer powder, polyethylene powder, polymethyl celsesquioxane powder, organopolysiloxane elastomer powder, urethane powder, wool powder, silk powder, crystalline cellulose, and N-acyllysine.

Examples of composite powders include reduced titanium natural mica powder coated with titanium oxide fine particles, titanium natural mica powder coated with zinc oxide fine particles, titanium natural mica powder coated with barium sulfate, titanium oxide-containing silicon dioxide powder, and zinc oxide-containing silicon dioxide powder.

Examples of dye powders include organic colorants, and those used in food products specified by the Japanese Food Sanitation Act, and in pharmaceuticals, quasi-medical products and cosmetics specified by the Japanese Pharmaceutical Affairs Act are preferable. Examples of organic colorants include dye, lake and organic pigment. Examples of organic colorants include yellow, blue, red, green, orange, brown, purple and black pigments as follows.

Examples of yellow pigments include Acid Yellow 23 defined by the Color Index Generic Name (hereinafter referred also to as "C.I.G.N') (Japanese official color code: Yellow No. 4. Parenthetical color codes provided below are also of the Japanese official color codes.) Food Yellow 3 (Yellow No. 5), Acid Yellow 73 (Yellow No. 202(1), Yellow No. 202(2)), Acid Yellow 3 (Yellow No. 203), Acid Yellow 40 (Yellow No. 402), Acid Yellow 1 (Yellow No. 403), Acid Yellow 36 (Yellow No. 406), and Acid Yellow 11 (Yellow No. 407).

Examples of blue pigments include Blue No. 1, Blue No. 2, Blue No. 202, Blue No. 203, and Blue No. 205.

Examples of red pigments include Red No. 2, Red No. 3, Red No. 102, Red No. 104(1), Red No. 105(1), Red No. 106, Red No. 201, Red No. 202, Red No. 227, Red No. 230(1), Red No. 230(2), Red No. 231, Red No. 232, Red No. 401, Red No. 502, Red No. 503, Red No. 504, and Red No. 506.

Examples of green pigments include Green No. 3, Green No. 201, Green No. 204, Green No. 205, Green No. 401, and Green No. 402.

Examples of orange pigments include Orange No. 205, Orange No. 207, and Orange No. 402.

Examples of brown pigments include Brown No. 201.

Examples of purple pigments include Purple No. 401.

Examples of black pigments include Black No. 401.

The cosmetic composition of the present invention may further contain a surfactant, an antioxidant, perfume, a preservative, water, polyhydric alcohol such as glycerin and 1,3-butylene glycol, lower alcohol or a beauty component as needed.

(Embodiment 3)

In Embodiment 3, one example of the ink composition of the present invention will be described.

The ink composition of the present invention contains the bright pigment of the present invention, preferably the bright pigment of the present invention and a vehicle. Further, the ink composition of the present invention preferably includes a pigment such as an organic colorant because design-rich printing surfaces having a glittering vivid color can be achieved. Examples of the ink composition include inks for writing instruments such as various ballpoint pens and marking pens and printing inks such as gravure inks and offset inks. Further, the ink composition of the present invention can be employed in printing, for example, packages, wrapping paper, wallpaper, clothes, panels, textiles, various films and labels.

Examples of vehicles for inks for writing instruments include a mixture obtained by mixing a solvent with at least one selected from the group consisting of microbe polysaccharides and water-soluble vegetable polysaccharides. Examples of microbe polysaccharides include acrylic resin, styrene-acrylic copolymer, polyvinyl alcohol, polyacrylate, acryl-vinyl acetate copolymer, and xanthan gum. Examples of water-soluble vegetable polysaccharides include guar gum. Examples of solvents include water, alcohol, hydrocarbons, and esters.

Examples of vehicles for gravure inks include a mixture obtained by mixing a solvent with a resin. Examples of resins include: at least one selected from the group consisting of gum rosin, wood rosin, talloil rosin, lime rosin, rosin ester, maleate resin, polyamide resin, vinyl resin, nitrocellulose, cellulose acetate, ethyl cellulose, chlorinated rubber, cyclized rubber, ethylene-vinyl acetate copolymer resin, urethane resin, polyester resin, alkyd resin, gilsonite, dammar and shellac; water-soluble resin obtained by imparting water solubility to one selected from the mentioned resin group; and oil-in-water (O/W) type resins. Examples of solvents include hydrocarbon, alcohol, ether, ester, and water.

Examples of vehicles for offset inks include a mixture obtained by mixing a solvent with a resin such as rosin-modified phenolic resin, petroleum resin or alkyd resin. Example of solvents include plant oils such as linseed oil, tung oil and soybean oil, n-paraffin, isoparaffin, aromatic, naphthene, $\alpha$-olefin and water.

Each of the vehicles mentioned may further contain a dye, a pigment other than the bright pigment of the present invention, a surfactant, a lubricant, an antifoaming agent, a leveling agent, etc.

Although the content of each component of the ink composition varies depending how the ink composition is used, the present invention does set limits thereto and the content may be similar to that of conventionally known inks.

The ink composition of the present invention has the effect of reflecting near-infrared radiation because it contains the bright pigment of the present invention. By irradiating a printed matter obtained by the application of the ink composition of the present invention with light in the near-infrared region and detecting the presence or absence of reflection or the reflectivity, whether the printed matter is counterfeited or not can be identified. Thus, the ink composition of the present invention can be used as, for example, an identification ink composition.

(Embodiment 4)

In Embodiment 4, one example of the paint composition of the present invention will be described.

The paint composition of the present invention contains the bright pigment of the present invention, preferably the bright pigment of the present invention, a resin and a solvent. The paint composition may further include a curing agent as needed. The paint composition of the present invention preferably contains a colorant such as an organic colorant because design-rich painting surfaces having a glittering vivid color can be achieved. Examples of areas in which the paint composition of the present invention can be employed include: the exterior of automobiles, motorcycles and bicycles; the exterior of construction materials, tiles and furniture; household electrical appliances such as air conditioners, refrigerators and rice steamers; containers; office supplies; and sporting goods such as golf clubs, golf balls and tennis rackets.

Examples of the resin include: thermosetting resins such as acrylic resin, polyester resin, epoxy resin, phenol resin, urea resin, fluorine resin, polyester-urethane curable resin, epoxy-polyester curable resin, acrylic-polyester based resin, acrylic-urethane curable resin, acrylic-melamine curable resin, and polyester-melamine curable resin; and thermoplastic resins such as polyethylene resin, polypropylene resin, vinyl chloride resin, petroleum resin, thermoplastic polyester resin, and thermoplastic fluorine resin.

Examples of the solvent include: aliphatic hydrocarbons (e.g., hexane, heptane, octane); esters (e.g., ethyl acetate, n-butyl acetate, isobutyl acetate, n-butyl acetate); ketones (e.g., acetone, methyl ethyl ketone, methyl isobutyl ketone); ethers (e.g., diethyl ether, dioxane, tetrahydrofuran); cellosolves (e.g., methyl cellosolve (ethylene glycol monomethyl ether), ethyl cellosolve, propyl cellosolve, butyl cellosolve, phenyl cellosolve, benzyl cellosolve); and carbitols (e.g., diethylene glycol monomethyl ether, carbitol (diethylene glycol monoethyl ether), diethylene glycol monopropyl ether). They may be used alone or in a combination of two or more.

Examples of the curing agent include polyisocyanate, amine, polyamide, polybasic acid, acid anhydride, polysulfide, trifluoroboric acid, acid dihydrazide, and imidazole.

The content of the bright pigment of the present invention is adjusted to be preferably 0.1 to 30 mass%, and more preferably 1 to 20 mass% of the ink composition in a cured and dried coating film. This is because it is possible to impart adequate brightness to the coating film and to make the most of the background color.

(Embodiment 5)

In Embodiment 5, one example of the thermal insulation paint composition of the present invention will be described.

The thermal insulation paint composition of the present invention contains the bright pigment of the present invention. Because the thermal insulation paint composition of the present invention contains the bright pigment of the present invention, it shows high luminance and chroma and has the effect of reflecting near infrared radiation. Paint compositions with a reflectivity of 20% or more, preferably 25% or more, and more preferably 30% or more in the near-infrared region of 850 nm to 1950 nm fall within the scope of the thermal insulation composition of the present invention. With the application of the thermal insulation composition of the present invention to the roof and outer wall of constructions such as buildings and houses or to the exterior of vehicles such as automobiles, light in the near-infrared region contained in the sunlight can be reflected. As a result, it is possible to prevent the entry of thermal energy into the constructions and vehicles, and preferably an increase in the temperature inside the constructions and vehicles.

The thermal insulation paint composition of the present invention preferably contains the bright pigment of the present invention, a resin and a solvent. The thermal insulation paint composition may further contain a curing agent as needed. Further, the thermal insulation paint composition according to this embodiment may be of a curable type that contains a thermosetting resin as the main ingredient or of a varnish type that contains a thermoplastic resin as the main ingredient. In this embodiment, examples of "curable type" paint compositions include those employed in an application method where a paint composition is applied and then heated to cause cross-linking of the thermosetting resin to form a coating film. When the thermal insulation composition is of a curable type, it contains a thermosetting resin, a curing agent and/or a catalyst and a solvent. The same thermosetting resin, curing agent and solvent as the paint composition of Embodiment 4 can be used. In this embodiment, examples of "varnish type" paint compositions include those employed in an application method where a paint composition is applied and then the solvent is vaporized to form a coating film. When the thermal insulation composition is of a varnish type, it contains a thermoplastic resin and a solvent. The same thermoplastic resin and solvent as the paint composition of Embodiment 4 can be used.

The thermal insulation paint composition of the present invention preferably contains a pigment such as an organic colorant because design-rich painting surfaces having a glittering vivid color can be obtained. Examples of areas in which the thermal insulation paint composition of the present invention can be employed include: the exterior of automobiles, motorcycles and bicycles; the exterior of construction materials, tiles and furniture; household electrical appliances such as air conditioners, refrigerators and rice steamers; containers; office supplies; sporting goods such as golf clubs, golf balls and tennis rackets.

(Embodiment 6)

In Embodiment 6, one example of the resin composition of the present invention will be described.

The resin composition of the present invention contains the bright pigment of the present invention, preferably the bright pigment of the present invention and a base resin. The resin composition may further contain a curing agent as needed. Further, the resin composition of the present invention preferably contains a colorant such as an organic colorant because design-rich resin molded products having a glittering vivid color can be achieved. Examples of molded products made using the resin composition of the present invention include cosmetic containers, food containers, wall materials, floor materials, cases for household electrical appliances, accessories, stationeries, toys, bathtubs, bath equipment, shoes, sporting goods and toilet goods.

The same thermosetting resin or thermal plastic resin as that contained in the above described paint composition can be used as the base resin. Particularly, it is preferable to use a thermoplastic resin compatible with injection molding employed in molding molded products having a complex shape.

The inorganic base constituting the bright pigment of the present invention is preferably glass without cleavage like mica. This is because it can maintain the same particle size before and after injection molding.

The resin composition of the present invention may further contain a pigment other than the bright pigment of the present invention, a surfactant, a lubricant, an antifoaming agent, a leveling agent, etc.

The content of each component of the resin composition may be the same as conventionally known resin compositions and can be set appropriately in response to the type of molded product. In terms of improving the color of molded products made using the resin composition, the content of the bright pigment of the present invention is preferably 0.01 to 10 mass%, and more preferably 0.5 to 5 mass% of the resin composition.

Hereinafter, an artificial marble molded product will be described as one example of the resin composition of the present invention.

An artificial marble molded product preferably has a three-layer structure composed of a transparent gelcoat layer disposed at the top, an intermediate layer containing the bright pigment of the present invention and disposed below the transparent gelcoat layer, and an artificial marble layer containing a colored aggregate and disposed below the intermediate layer. Since the top layer of such artificial marble is a transparent gelcoat layer, the appearances of the layers lower than the gelcoat layer are reflected as the appearance of the artificial marble. Thus, the artificial marble molded product has glitter provided by the bright pigment contained in the intermediate layer and clearly exhibits a marble-like appearance.

The transparent gelcoat layer preferably has a thickness of 0.3 mm to 0.7 mm, considering the sense of depth and a reduction in visible light transmittance. Although the composition of the transparent gelcoat layer is not particularly limited, a thermosetting resin is preferable because of its ease of handling and having high process moldability. Specifically, examples of the thermosetting resin include unsaturated polyester resin, vinyl ester resin, epoxy resin, urethane resin, phenol resin, or a mixture or a modified product (e.g., a modified product obtained by changing the end group of unsaturated polyester resin to acrylic) thereof. An unsaturated polyester resin is particularly preferable because of its high transparency, cheapness and availability.

The intermediate layer is for imparting high brightness to the marble-like appearance and it should not cover the pattern of the artificial marble layer. Therefore, the intermediate layer needs to have at least visible light transmittance, but its main ingredient need not be the same as that of the top transparent gelcoat.

As long as the appearance is not compromised, layers other than the intermediate layer may be further disposed between the transparent gelcoat layer and the artificial marble layer. For example, in addition to the intermediate layer, a colored film having high visible light transmittance may be disposed between the transparent gelcoat layer and the artificial marble layer. In this case, the color of the artificial marble molded product can be easily adjusted.

The intermediate layer preferably has a thickness of 0.05 mm to 1 mm. A thermosetting resin with high visible light transmittance is desirable as a component of the intermediate layer. Further, the intermediate layer may further contain a curing agent and an accelerator, in addition to the pigment of the present invention. The intermediate layer may contain a thickener, a thixotropic agent, an antifoaming agent or a property improver as needed. Furthermore, the intermediate layer may contain one or more pigments selected from the group consisting of a coloring pigment, other metallic pigment (aluminum pigment and iron-oxide pigment) and an interference color pigment (mica coated with a metal oxide, etc.) within the bounds of not compromising the background color.

The artificial marble layer preferably has a thickness of 3 mm to 25 mm. The artificial marble layer contains a thermosetting resin as its main ingredient and may contain an aggregate, a promoter, a curing agent and a colorant as other ingredients. For the thermosetting resin, the same thermosetting resin as that used in the transparent gelcoat layer may be used and examples of which include unsaturated polyester resin. Examples of the aggregate include: inorganic materials such as glass frit, white marble, aluminium hydroxide, calcium carbonate and silica powder; and organic materials such as thermoplastic polyester resin. The artificial marble layer may further contain glass fibers as a reinforcement as needed.

The present invention does not set limits to the content of each ingredient of each layer, and the content may be similar to that of conventionally known artificial marble molded products. However, the content of the bright pigment in the intermediate layer is preferably 0.01 to 10 parts by mass per 100 parts by mass of the thermosetting resin because appropriate properties, high brightness and adequate sense of depth can be achieved.

When high strength is required of the artificial marble molded product, a fiber reinforced plastic (herein after referred to as "FRP") layer may be provided on the backside (the surface opposite to the surface facing the intermediate layer) of the artificial marble layer. Because the FRP layer is to be disposed on the backside of the artificial marble layer, it does not compromise the brightness of the artificial marble molded product. The FRP layer is obtained by, for example, impregnating chopped strand glass mat with a thermosetting resin, such as an unsaturated polyester resin, a vinyl ester resin or an epoxy resin, and heat-curing them. Further, by adding a colorant to the FRP layer, a color can be applied to the artificial marble molded product. This allows blockage of light beams that enter from the backside.

The method for producing the artificial marble molded product is not particularly limited and a conventional method can be used in its entirety. For example, when producing a product with a complex shape such as a bathtub, casting, press molding or the like can be used. Further, when producing a bathtub including the FRP layer, the following methods can be used. In one method (spray-up), the artificial marble layer is formed, and then a thermosetting resin containing chopped strand glass is sprayed onto the surface of the artificial marble layer with a spray gun. In another method, a chopped strand glass mat impregnated with a thermosetting resin is mounted on the surface of the artificial marble layer by hand lay-up, and they are cured for an hour at 50°C. Or, prior to forming the artificial marble layer, the FRP layer may be pre-formed on the surface of the mold release material of the bathtub by spray-up or hand lay-up.

When the inorganic base constituting the bright pigment is scaly glass, the bright pigment preferably has an average particle size of 20 $\mu$m to 200 $\mu$m and an average thickness of 0.5 $\mu$m to 7 $\mu$m. When the average particle size and average thickness of the bright pigment are respectively within the ranges mentioned, the bright pigment becomes less like to be shattered in the course of forming the artificial marble molded product. Thus, the artificial marble molded product having adequate brightness can be formed.

(Embodiment 7)

In Embodiment 7, one example of the coated paper of the present invention will be described.

The coated paper can be obtained by coating one or both surfaces of base paper with a single or two or more layers of a solution containing an adhesive, the bright pigment of the present invention, a surfactant and a solvent. At least one of an antioxidant, a UV absorber, a waterproofing agent, an antiseptic-antifungal agent, a germicide, an antifoaming agent, perfume and a pigment may be added to the solution as needed. Examples of the adhesive include stearic acid, an alkali salt of lauryl acid, copolymerized latex, and starch.

The coating can be carried out with a coater, such as a blade coater, an air knife coater, a roll coater, a curtain coater, a bar coater, a gravure coater and a size press coater.

When the inorganic base constituting the bright pigment is scaly glass, the bright pigment preferably has an average particle size of 20 $\mu$m to 150 $\mu$m and an average thickness of 0.2 $\mu$m to 3 $\mu$m. When the average particle size and average thickness of the bright pigment are respectively within the ranges mentioned, the coated paper having surface smoothness and adequate brightness can be formed.

Examples of the colorants mentioned in Embodiments 3 to 7 include yellow, red, blue, green, orange and black organic colorants as follows.

Examples of yellow pigments include Pigment Yellow 1, Pigment Yellow 3, Pigment Yellow 12, Pigment Yellow 13, Pigment Yellow 14, Pigment Yellow 16, Pigment Yellow 17, Pigment Yellow 42, Pigment Yellow 53, Pigment Yellow 73, Pigment Yellow 74, Pigment Yellow 81, Pigment Yellow 83, Pigment Yellow 97, Pigment Yellow 102, Pigment Yellow 111, Pigment Yellow 120, Pigment Yellow 126, Pigment Yellow 127, Pigment Yellow 139, Pigment Yellow 147, Pigment Yellow 151, Pigment Yellow 154, Pigment Yellow 155, Pigment Yellow 173, Pigment Yellow 174, Pigment Yellow 175, Pigment Yellow 176, Pigment Yellow 181, Pigment Yellow 184, Pigment Yellow 191, Pigment Yellow 192, Pigment Yellow 194, Pigment Yellow 196, Pigment Yellow 213, Pigment Yellow 214, Solv. Yellow 5, and Solv. Yellow 6 defined by C.I.G.N.

Examples of red pigments include Pigment Red 2, Pigment Red 3, Pigment Red 4, Pigment Red 5, Pigment Red 9, Pigment Red 12, Pigment Red 14, Pigment Red 38, Pigment Red 48:2, Pigment Red 48:3, Pigment Red 48:4, Pigment Red 52:2, Pigment Red 53:1, Pigment Red 57:1, Pigment Red 101, Pigment Red 112, Pigment Red 122, Pigment Red 144, Pigment Red 146, Pigment Red 147, Pigment Red 149, Solv. Red 49, Solv. Red 48, Pig. Red 64, Pig. Red 63(Ca), Solv. Red 43, Solv. Red 23, Vat Red 1, Pig. Red 4, Pig. Red 22, Pig. Red 48, Solv Red 24 and Solv Red 7 denned by C.I.G.N.

Examples of blue pigments include Pigment Blue 15, Pigment Blue 15:1, Pigment Blue 15:2, Pigment Blue 15: 3, Pigment Blue 15:4, Pigment Blue 16, and Pigment Blue 28 defined by C.I.G.N.

Examples of orange pigments include Pigment Orange 5, Pigment Orange 13, Pigment Orange 34, Pigment Orange 36, Pigment Orange 38, Pigment Orange 43, Pigment Orange 62, and Pigment Orange 68 defined by C.I.G.N.

Examples of green pigments include Pigment Green 7, Pigment Green 17, Pigment Green 36, and Pigment Green 50 defined by C.I.G.N.

Examples of black pigments include Pigment Black 7, Pigment Black 11 and Pigment Black 33 defined by C.I.G.N.

Examples

Hereinafter, the present invention will be described in more detail with reference to Examples and Comparative Examples.

The silver or silver alloy content in each bright pigment, the average thickness and average particle size of each inorganic base and those of each bright pigment and the average thickness of each silver-containing coating were measured as follows.

[Silver or Silver Alloy Content]

The silver or silver-alloy content in each bright pigment was determined by entirely dissolving each bright pigment in aqua regia and measuring the silver or silver alloy content with an inductively coupled plasma mass spectrometer (trade name: ICPS7500, manufactured by Shimadzu Corporation).

[Average Thickness of Inorganic Base]

The average thickness of each inorganic base was determined as follows. The thickness of each inorganic base was measured with an electron microscope (trade name: VE-7800, manufactured by Keyence Corporation) based on cross sections of 30 grains of each bright pigment, and an average thickness was determined.

[Average Thickness of Bright Pigment]

The average thickness of each bright pigment was determined as follows. For 60 grains of each bright pigment, their end face thickness (a in FIG.1) was measured with an electron microscope (trade name: VE-7800, manufactured by Keyence Corporation) and an average thickness was determined.

[Average Thickness of Silver-containing Coating]

The average thickness of each silver-containing coating was determined as follows. The thickness of each silver-containing coating was measured based on the cross section of each bright pigment (10 grains) observed with a field emission scanning electron microscope (FE-SEM) and an average thickness was determined. S-4800 (manufactured by Hitachi High-Technologies) was used as the field emission scanning electron microscope.

[Average Particle Size]

The average particle size of each inorganic base and each bright pigment was determined as follows. The particle size of each inorganic base and each bright pigment was measured with a laser diffraction particle size analyzer (trade name: Microtrack HRA, manufactured by Nikkiso CO., LTD.) and the particle size corresponding to a particle diameter at which the cumulative volume of the particles reached 50% in a particle size distribution (D50) was determined.

(Example 1)

A bright pigment of Example 1 was produced as follows.

[Scaly Inorganic Base]

A scaly glass base (average particle size: 32 $\mu$m, average thickness: 5.0 $\mu$m) was obtained by pulverizing a glass piece with a pulverizer (the glass piece had been formed from molten glass by ballooning), and classifying the thus pulverized scaly glass base with a vibration sieve, an ultrasonic sieve, and an airflow classifier.

[Pretreatment of Inorganic Base]

0.15 g of stannous chloride was added to 1 L of pure water and dissolved, to which dilute hydrochloric acid was added further to obtain a pretreatment solution for electroless plating (pH: 2.0 to 2.2). After adding 170 g of the scaly glass base to the obtained pretreatment solution, the scaly glass base was taken out from the pretreatment solution and rinsed with water. This process was repeated several times as a pretreatment of the scaly glass base.

[Formation of Silver-containing Coating]

First, 52.5 g of 25 mass% aqueous ammonia as a complexing agent, 36 ml of ethylene diamine, a pH adjuster and 28.5 g of silver nitrate as a silver material were added to 2 L of pure water, which then was stirred while being warmed to 30°C so as to obtain a plating solution A. A sodium hydroxide aqueous solution obtained by dissolving 5 g of sodium hydroxide in 30 ml of pure water was used as the pH adjuster. Meanwhile, 15 g of glucose as a reducing agent was added to 500 ml of pure water so as to obtain a solution, to which the pretreated scaly glass base was added and stirred to obtain a plating solution B.
Next, the plating solution B was added to the plating solution A, and the mixed solution was stirred for 20 minutes so as to deposit silver on the surface of the scaly glass base by an electroless plating reaction, thereby forming a silver-containing coating.
The scaly glass base coated with the silver-containing coating was filtered and rinsed with water for several times, followed by drying at 180°C. Finally, the scaly glass base coated with the silver-containing coating was subjected to a heat treatment for 2 hours at 400°C using an electronic muffle furnace, thus obtaining the bright pigment of Example 1. As for the bright pigment of Example 1, the inorganic base had an average thickness of 5.0 $\mu$m, the bright pigment had an average particle size of 35 $\mu$m, an average thickness of 5.1 $\mu$m, and a silver content of 8 mass%, and the silver-containing coating had an average thickness of 45 nm. The bright pigment exhibited a white metallic luster.

(Example 2)

A bright pigment of Example 2 was obtained in the same manner as Example 1 except that the amounts of the stannous chloride and the scaly glass base were changed to 0.2 g and 109 g, respectively. As for the bright pigment of Example 2, the inorganic base had an average thickness of 5.0 $\mu$m, the bright pigment had an average particle size of 35 $\mu$m, an average thickness of 5.1 $\mu$m, and a silver content of 12 mass%, and the silver-containing coating had an average thickness of 71 nm. The bright pigment exhibited a white metallic luster.

(Example 3)

A bright pigment of Example 3 was obtained in the same manner as Example 1 except that the amounts of the stannous chloride and the scaly glass base were changed to 0.1 g and 85 g, respectively. As for the bright pigment of Example 3, the inorganic base had an average thickness of 5.0 $\mu$m, the bright pigment had an average particle size of 35 $\mu$m, an average thickness of 5.2 $\mu$m, and a silver content of 15 mass%, and the silver-containing coating had an average thickness of 91 nm. The bright pigment exhibited a white metallic luster.

(Example 4)

A bright pigment of Example 4 was obtained in the same manner as Example 1 except that a scaly glass base having an average particle size of 32 $\mu$m and an average thickness of 1.3 $\mu$m was used and the amounts of the stannous chloride and the scaly glass base were changed to 0.4 g and 62 g, respectively. As for the bright pigment of Example 4, the inorganic base had an average thickness of 1.3 $\mu$m, the bright pigment had an average particle size of 35 $\mu$m, an average thickness of 1:4 $\mu$m, and a silver content of 19 mass%, and the silver-containing coating had an average thickness of 32 nm. The bright pigment exhibited a white metallic luster.

(Example 5)

A bright pigment of Example 5 was obtained in the same manner as Example 4 except that the amounts of the stannous chloride and the scaly glass base were changed to 0.3 g and 37 g, respectively As for the bright pigment of Example 5, the inorganic base had an average thickness of 1.3 $\mu$m, the bright pigment had an average particle size of 35 $\mu$m, an average thickness of 1.4 $\mu$m, and a silver content of 29 mass%, and the silver-containing coating had an average thickness of 53 nm. The bright pigment exhibited a white metallic luster.

(Example 6)

A bright pigment of Example 6 was obtained in the same manner as Example 4 except that the amounts of the stannous chloride and the scaly glass base were changed to 0.15 g and 23 g, respectively As for the bright pigment of Example 6, the inorganic base had an average thickness of 1.3 $\mu$m, the bright pigment had an average particle size of 35 $\mu$m, an average thickness of 1.5 $\mu$m, and a silver content of 40 mass%, and the silver-containing coating had an average thickness of 89 nm. The bright pigment exhibited a white metallic luster.

(Example 7)

A bright pigment of Example 7 was obtained in the same manner as Example 1 except that a scaly glass base having an average particle size of 32 $\mu$m and an average thickness of 0.7 $\mu$m was used and the amounts of the stannous chloride and the scaly glass base were changed to 0.5 g and 38 g, respectively. As for the bright pigment of Example 7, the inorganic base had an average thickness of 0.7 $\mu$m, the bright pigment had an average particle size of 35 $\mu$m, an average thickness of 0.8 $\mu$m, and a silver content of 28 mass%, and the silver-containing coating had an average thickness of 28 nm. The bright pigment exhibited a white metallic luster.

(Example 8)

A bright pigment of Example 8 was obtained in the same manner as Example 7 except that the amounts of the stannous chloride and the scaly glass base were changed to 0.3 g and 21 g, respectively As for the bright pigment of Example 8, the inorganic base had an average thickness of 0.7 $\mu$m, the bright pigment had an average particle size of 35 $\mu$m, an average thickness of 0.8 $\mu$m, and a silver content of 42 mass%, and the silver-containing coating had an average thickness of 52 nm. The bright pigment exhibited a white metallic luster.

(Example 9)

A bright pigment of Example 9 was obtained in the same manner as Example 7 except that the amounts of the stannous chloride and the scaly glass base were changed to 0.18 g and 12 g, respectively. As for the bright pigment of Example 9, the inorganic base had an average thickness of 0.7 $\mu$m, the bright pigment had an average particle size of 35 $\mu$m, an average thickness of 0.9 $\mu$m, and a silver content of 55 mass%, and the silver-containing coating had an average thickness of 89 nm. The bright pigment exhibited a white metallic luster.

(Example 10)

A bright pigment of Example 10 was obtained in the same manner as Example 1 except that the amount of the stannous chloride was changed to 0.4 g and 28 g of a scaly synthetic mica base (product name: PDM-40L, manufactured by Topy Industries Ltd, average particle size: 40 $\mu$m, average thickness: 0.4 $\mu$m) was used in place of the scaly glass base. As for the bright pigment of Example 10, the inorganic base had an average thickness of 0.4 $\mu$m, the bright pigment had an average particle size of 42 $\mu$m, an average thickness of 0.4 $\mu$m, and a silver content of 35 mass%, and the silver-containing coating had an average thickness of 22 nm. The bright pigment exhibited a white metallic luster.

(Example 11)

A bright pigment of Example 11 was obtained in the same manner as Example 10 except that the amounts of the stannous chloride and the scaly synthetic mica base were changed to 0.3 g and 15 g, respectively. As for the bright pigment of Example 11, the inorganic base had an average thickness of 0.4 $\mu$m, the bright pigment had an average particle size of 42 $\mu$m, an average thickness of 0.5 $\mu$m, and a silver content of 50 mass%, and the silver-containing coating had an average thickness of 41 nm. The bright pigment exhibited a white metallic luster.

(Example 12)

A bright pigment of Example 12 was obtained in the same manner as Example 10 except that the amounts of the stannous chloride and the scaly synthetic mica base were changed to 0.15 g and 7 g, respectively. As for the bright pigment of Example 12, the inorganic base had an average thickness of 0.4 $\mu$m, the bright pigment had an average particle size of 42 $\mu$m, an average thickness of 0.6 $\mu$m, and a silver content of 68 mass%, and the silver-containing coating had an average thickness of 87 nm. The bright pigment exhibited a white metallic luster.

(Comparative Example 1)

A bright pigment of Comparative Example 1 was obtained in the same manner as Example 1 except that the amounts of the stannous chloride and the scaly glass base were changed to 1.0 g and 720 g, respectively As for the bright pigment of Comparative Example 1, the inorganic base had an average thickness of 5.0 $\mu$m, the bright pigment had an average particle size of 35 $\mu$m, an average thickness of 5.0 $\mu$m, and a silver content of 2 mass%, and the silver-containing coating had an average thickness of 11 nm. The bright pigment exhibited a dark gray metallic luster.

(Comparative Example 2)

A bright pigment of Comparative Example 2 was obtained in the same manner as Example 1 except that the amounts of the stannous chloride and the scaly glass base were changed to 0.1 g and 60 g, respectively. As for the bright pigment of Comparative Example 2, the inorganic base had an average thickness of 5.0 $\mu$m, the bright pigment had an average particle size of 45 $\mu$m, an average thickness of 5:3 $\mu$m, and a silver content of 20 mass%, and the silver-containing coating had an average thickness of 128 nm. The bright pigment exhibited a metallic luster with less white glitter.

(Comparative Example 3)

A bright pigment of Comparative Example 3 was obtained in the same manner as Example 4 except that the amounts of the stannous chloride and the scaly glass base were changed to 0.8 g and 110 g, respectively. As for the bright pigment of Comparative Example 3, the inorganic base had an average thickness of 1.3 $\mu$m, the bright pigment had an average particle size of 35 $\mu$m, an average thickness of 1.3 $\mu$m, and a silver content of 12 mass%, and the silver-containing coating had an average thickness of 18 nm. The bright pigment exhibited a dark gray metallic luster.

(Comparative Example 4)

A bright pigment of Comparative Example 4 was obtained in the same manner as Example 4 except that the amounts of the stannous chloride and the scaly glass base were changed to 0.12 g and 15 g, respectively. As for the bright pigment of Comparative Example 4, the inorganic base had an average thickness of 1.3 $\mu$m, the bright pigment had an average particle size of 35 $\mu$m, an average thickness of 1.6 $\mu$m, and a silver content of 50 mass%, and the silver-containing coating had an average thickness of 133 $\mu$m. The bright pigment exhibited a metallic luster with less white glitter.

(Comparative Example 5)

A bright pigment of Comparative Example 5 was obtained in the same manner as Example 7 except that the amounts of the stannous chloride and the scaly glass base were changed to 1.0 g and 73 g, respectively As for the bright pigment of Comparative Example 5, the inorganic base had an average thickness of 0.7 $\mu$m, the bright pigment had an average particle size of 35 $\mu$m, an average thickness of 0.7 $\mu$m, and a silver content of 17 mass%, and the silver-containing coating had an average thickness of 15 nm. The bright pigment exhibited a dark gray metallic luster.

(Comparative Example 6)

A bright pigment of Comparative Example 6 was obtained in the same manner as Example 7 except that the amounts of the stannous chloride and the scaly glass base were changed to 0.12 g and 8 g, respectively. As for the bright pigment of Comparative Example 6, the inorganic base had an average thickness of 0.7 $\mu$m, the bright pigment had an average particle size of 35 $\mu$m, an average thickness of 1.0 $\mu$m, and a silver content of 65 mass%, and the silver-containing coating had an average thickness of 133 nm. The bright pigment exhibited a metallic luster with less white glitter.

(Comparative Example 7)

A bright pigment of Comparative Example 7 was obtained in the same manner as Example 10 except that the amounts of the stannous chloride and the scaly synthetic mica base were changed to 1.3 g and 51 g, respectively. As for the bright pigment of Comparative Example 7, the inorganic base had an average thickness of 0.4 $\mu$m, the bright pigment had an average particle size of 42 $\mu$m, an average thickness of 0.4 $\mu$m, and a silver content of 22 mass%, and the silver-containing coating had an average thickness of 12 nm. The bright pigment exhibited a dark gray metallic luster.

(Comparative Example 8)

A bright pigment of Comparative Example 8 was obtained in the same manner as Example 10 except that the amounts of the stannous chloride and the scaly synthetic mica base were changed 0.08 g and 4.2 g, respectively. As for the bright pigment of Comparative Example 8, the inorganic base had an average thickness of 0.4 $\mu$m, the bright pigment had an average particle size of 42 $\mu$m, an average thickness of 0.7 $\mu$m, and a silver content of 78 mass%, and the silver-containing coating had an average thickness of 147 nm. The bright pigment exhibited a metallic luster with less white glitter.

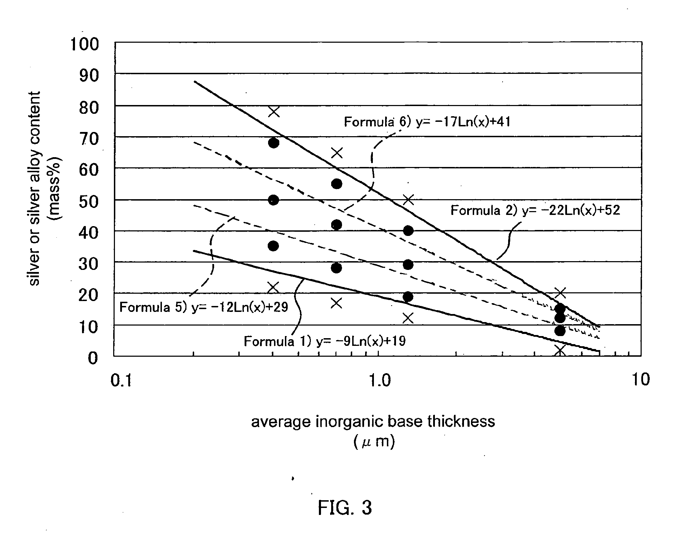

While the bright pigments of Examples 1 to 12 satisfied the formulas 1) and 2), those of Comparative Examples 1 to 8 did not. FIG. 3 shows the relationship between the organic base average thickness and the silver content of the bright pigments of Examples 1 to 12 and Comparative Examples 1 to 8 as well as the formulas 1), 2), 5) and 6). In FIG. 3, black circles indicate Examples and crosses indicate Comparative Examples. Further, in FIG. 3, the formulas 1) and 2) are indicated by solid lines and the formulas 5) and 6) are indicated by broken lines.

Next, each of the obtained bright pigments of Examples 1 to 12 and those of Comparative Examples 1 to 8 was each used in producing nail varnish, and the following evaluations were made.

[Production of Nail Varnish]

100 parts by mass of a nail varnish composition shown in Table 1 and 5 parts by mass of each of the bright pigments of Examples 1 to 12 and Comparative Examples 1 to 8 were mixed with each other using a kneading machine to obtain a total of 20 nail varnishes.

[TABLE 1]

| Nail varnish composition | Parts by mass |
|---|---|
| isopropanol (IPA) | 5 |
| ethyl acetate | 21 |
| nitrocellulose (30 mass%, including IPA) | 11 |
| stearyl benzyl dimethyl ammonium modified hectorite | 1.5 |
| acetyl tributyl citrate | 1.8 |
| N-ethyl-O,P- toluenesulfonamide | 2.6 |
| alkyd resin (including 30 mass% of ethyl acetate) | 12 |
| butyl acetate | 45 |
| citric acid 1/1 $H_2O$ | 0.1 |
| Total | 100 |

[Production of Cosmetic Evaluation Sample]

Each nail varnish was applied onto a commercially available hiding chart (Munsell N1) such that the nail varnish thickness reached 9 mil (0.23 mm), followed by air-drying, and thus obtaining cosmetic evaluation samples. Further, as a reference example, a paint only containing 100 parts by mass of the nail varnish composition (containing no bright pigment) was produced, and a cosmetic evaluation sample was obtained in the same manner.

[Optical Evaluation]

For each cosmetic evaluation sample, its lightness L*, chroma C*, and luminance were obtained as follows. Table 2 provides the results.

Lightness L*, Chroma C*

The lightness (value of L*) and chroma (values of a* and b*) of each cosmetic evaluation sample were measured with a colorimeter (CR-400, manufactured by Konica Minolta Sensing Inc.) using an observation light source: illuminant C and a L*a*b* color system. The chroma C* was determined by calculation using the following formula:

$$\text{Chroma } C^* = \{(a^*)^2 + (b^*)^2\}^{1/2}.$$

Luminance

The luminance ($cd/m^2$) was measured with a luminance meter (LS-100, manufactured by Konica Minolta Sensing Inc.). As shown in FIG. 2, light reflected from a coating film 50 was received by a luminance meter 30 to measure the luminance. The luminance meter 30 was disposed so as to be perpendicular to the coating film 50. A light source 40 was disposed so that irradiated light and light reflected from the coating film 50 (incident light to the luminance meter 30) formed an angle of 5°.

[Sensory Evaluation]

20 female participants were each asked to actually use all of the 20 nail varnishes (nail varnishes containing the bright pigments of Examples 1 to 12 and Comparative Examples 1 to 8, respectively) for four weeks to conduct sensory evaluations on the use characteristic (the cosmetic effect of having vivid red glitter) of each nail varnish. Table 2 provides the results. Criteria for evaluating the cosmetic effect are as follows.

Criteria for Evaluating Cosmetic Effect

Very effective: the number of the participants who rated nail varnish as having a high cosmetic effect was 15 or more

Effective: the number of the participants who rated nail varnish as having a high cosmetic effect was 10 to 14

Somewhat effective: the number of the participants who rated nail varnish as having a high cosmetic effect was 7 to 9

Not effective: the number of the participants who rated nail varnish as having a high cosmetic effect was 6 or less

[TABLE 2]

| | Inorganic base | | Bright pigment | | | Cosmetic evaluation | | | |
|---|---|---|---|---|---|---|---|---|---|
| | Material | Average Thickness ($\mu$m) | Silver or silver alloy content (mass%) | Average thickness of silver containing coating (nm) | Luster | Lightness L* | Chroma C* | Luminance (cd/m²) | Cosmetic effect |
| Ex.1 | glass | 5.0 | 8 | 45 | white luster | 47 | 26 | 16700 | somewhat effective |
| Ex.2 | glass | 5.0 | 12 | 71 | white luster | 51 | 33 | 17800 | somewhat effective |
| Ex.3 | glass | 5.0 | 15 | 91 | white luster | 48 | 31 | 17000 | somewhat effective |
| Ex.4 | glass | 1.3 | 19 | 32 | white luster | 53 | 26 | 17200 | effective |
| Ex.5 | glass | 1.3 | 29 | 53 | white luster | 58 | 35 | 18800 | effective |
| Ex.6 | glass | 1.3 | 40 | 89 | white luster | 54 | 33 | 17500 | effective |
| Ex.7 | glass | 0.7 | 28 | 28 | white luster | 56 | 29 | 18100 | very effective |
| Ex.8 | glass | 0.7 | 42 | 52 | white luster | 59 | 36 | 19000 | very effective |
| Ex.9 | glass | 0.7 | 55 | 89 | white luster | 54 | 33 | 18500 | very effective |
| Ex.10 | synthetic mica | 0.4 | 35 | 22 | white luster | 51 | 27 | 19200 | very effective |
| Ex.11 | synthetic mica | 0.4 | 50 | 41 | white luster | 52 | 37 | 19500 | very effective |
| Ex.12 | synthetic mica | 0.4 | 68 | 87 | white luster | 47 | 32 | 19000 | very effective |
| Comp. Ex.1 | glass | 5.0 | 2 | 11 | dark gray | 27 | 19 | 8700 | not effective |
| Comp. Ex.2 | glass | 5.0 | 20 | 128 | luster with less white glitter | 35 | 24 | 10500 | not effective |
| Comp. Ex.3 | glass | 1.3 | 12 | 18 | dark gray | 45 | 20 | 11000 | not effective |
| Comp. Ex.4 | glass | 1.3 | 50 | 133 | luster with less white glitter | 38 | 25 | 11800 | not effective |
| Comp. Ex5 | glass | 0.7 | 17 | 15 | dark gray | 41 | 19 | 11500 | not effective |

(continued)

| | Inorganic base | | Bright pigment | | | Cosmetic evaluation | | | |
|---|---|---|---|---|---|---|---|---|---|
| | Material | Average Thickness ($\mu$m) | Silver or silver alloy content (mass%) | Average thickness of silver containing coating (nm) | Luster | Lightness L* | Chroma C* | Luminance (cd/m$^2$) | Cosmetic effect |
| Comp. Ex.6 | glass | 0.7 | 65 | 133 | luster with less white glitter | 34 | 24 | 11000 | not effective |
| Comp. Ex. 7 | synthetic mica | 0.4 | 22 | 12 | dark gray | 45 | 21 | 13100 | not effective |
| Comp. Ex.8 | synthetic mica | 0.4 | 78 | 147 | luster with less white glitter | 41 | 25 | 13800 | not effective |
| Ref: Ex. | - | - | - | - | - | 24 | 76 | 1800 | - |

As can be seen from Table 2 and FIG. 3, as to the nail varnishes that respectively contained the bright pigments of Examples 1 to 12 having an average inorganic base thickness of 0.2 μm to 7.0 μm and a silver or silver alloy content satisfying the formulas 1) and 2), they had higher luminance than any of the nail varnishes that respectively contained the bright pigments of Comparative Examples 1 to 8 and showed favorable results on lightness and chroma. Further, the bright pigments of Examples 1 to 12 having a silver or silver alloy content satisfying the formula 1) and 2) all exhibited a luster with excellent white brightness. On the other hand, the bright pigments of Comparative Examples 1, 3 and 5, which had a silver or silver alloy content less than the content obtained from the formula 1), showed a dark gray luster. Further, the bright pigments of Comparative Examples 2, 4, 6 and 8, which had a silver or silver alloy content larger than the content obtained from the formula 2), showed a luster with less white brightness. It is assumed that one of the reasons why the bright pigments of Comparative Examples 2, 4, 6 and 8 exhibited a luster with less white brightness is because the silver or silver alloy content to the inorganic base became excessive, so that silver agglomerated and asperities on the coating film surface increased in size, causing a decline in luminance.

As for the nail varnishes that respectively contained the bright pigments of Examples 7 to 12 having an average inorganic base thickness of 0.2 μm or more and less than 1.0 μm, asperities on the coating film became smaller. This resulted in high lightness, chroma and luminance and their cosmetic effect were rated as favorable in the sensory evaluations.

[Production of Thermal Insulation Paint Composition]

Vinyl chloride varnish shown in Table 3 and each of twelve bright pigments shown in Table 5 (the bright pigments of Examples 4, 6, 7, 9, 10 and 12 and Comparative Examples 3 to 8) were mixed with each other using a kneading machine to obtain a total of 12 thermal insulation paint compositions (Examples 13 to 18 and Comparative Examples 9 to 14) shown in Table 5.

[TABLE 3]

| Thermal insulation paint composition | Content weight |
|---|---|
| vinyl chloride resin varnish (Vinylose Clear* manufactured by Dai Nippon Thryo Co., LTD.) | 45g |
| Bright pigment | 0.25 g |
| *Vinylose Clear contains toluene (5 to 10%), xylene (30 to 40%) and butyl acetate (10 to 20%). | |

[Production of Thermal Insulation Evaluation Sample]

Each thermal insulation paint composition was applied onto a commercially available PET film (thickness: 100 μm) such that the composition thickness reached 9 - mil (0.23 mm), followed by air-drying, and thus obtaining thermal insulation evaluation samples. Further, a paint only containing the vinyl chloride resin varnish (containing no bright pigment) was produces. A reference sample for measuring near-infrared spectroscopy (described later) was obtained in the same manner as the thermal insulation evaluation samples except that only the vinyl chloride resin varnish was used in place of the thermal insulation paint composition.

[Optical Evaluation]

Using each thermal insulation evaluation sample and the reference sample, the near-infrared diffuse reflectivity of each thermal insulation evaluation sample in a wavelength range of 830 nm to 2000 nm was measured. A near-infrared ultraviolet and visible spectrophotometer equipped with an integrating sphere unit (UV-3600, manufactured by Shimadzu Corporation) was used to measure each reflectivity. Table 5 provides the results. Criteria for evaluating the thermal insulation effect in Table 5 are as shown in Table 4.

[TABLE 4]

| Criteria for Evaluating Thermal Insulation Effect | | | |
|---|---|---|---|
| | Near infrared reflectivity | | |
| | 850 nm | 1400 nm | 1950 nm |
| Very effective | 23% or more | 28% or more | 35% or more |
| Effective | 15% or more and less than 23% | 22% or more and less than 28% | 30% or more and less than 35% |
| Not Effective | less than 15% | less than 22% | less than 30% |

[TABLE 5]

| | | Bright pigment | | | | Optical evaluation | | | |
|---|---|---|---|---|---|---|---|---|---|
| | | Inorganic base | | Silver or silver alloy content (mass%) | Average thickness of silver containing coating | Near infrared reflectivity (%) | | | |
| | | Material | Average thickness ($\mu$m) | | | 850 (nm) | 1400 | 1950 | Thermal insulation effect |
| Ex.13 | Ex. 4 | glass | 1.3 | 19 | 32 | 201 | 24.5 | 33.1 | effective |
| Ex.14 | Ex. 7 | glass | 0.7 | 28 | 28 | 25.6 | 29.3 | 37.9 | Very effective |
| Ex.15 | Ex. 10 | synthetic mica | 0.4 | 35 | 22 | 31.4 | 36.8 | 42.6 | Very effective |
| Ex.16 | Ex. 6 | glass | 1.3 | 40 | 89 | 19.5 | 23.3 | 32.4 | effective |
| Ex.17 | Ex. 9 | glass | 0.7 | 55 | 89 | 23.9 | 28 | 36.5 | Very effective |
| Ex.18 | Ex. 12 | synthetic mica | 0.4 | 68 | 87 | 30 | 35.9 | 40.3 | Very effective |
| Comp. Ex.9 | Comp. Ex. 3 | glass | 1.3 | 12 | 18 | 10.8 | 14.6 | 22.2 | Not effective |
| Comp. Ex.10 | Comp. Ex.5 | glass | 0.7 | 17 | 15 | 11.8 | 16.1 | 24.4 | Not effective |
| Comp. Ex.11 | Comp. Ex. 7 | synthetic mica | 0.4 | 22 | 12 | 12.7 | 18.2 | 26.3 | Not effective |
| Comp. Ex.12 | Comp. Ex. 4 | glass | 1.3 | 50 | 133 | 11.5 | 16.7 | 25.3 | Not effective |
| Comp. Ex.13 | Comp. Ex. 6 | glass | 0.7 | 65 | 133 | 12.6 | 18.4 | 27.6 | Not effective |
| Comp. Ex.14 | Comp. Ex.8 | synthetic mica | 0.4 | 78 | 147 | 13.4 | 20.2 | 29.1 | Not effective |

As shown in Table 5, as to the thermal insulation paint compositions of Examples 13 to 18 that respectively contained the bright pigments of Examples 4, 6, 7, 9, 10 and 12 having an average inorganic base thickness of 0.2 $\mu$m to 7.0 $\mu$m and a silver or silver alloy content satisfying the formulas 1) and 2), they had a near-infrared reflectivity higher than any of the thermal insulation paint compositions of Comparative Examples 9 to 14 that respectively contained the bright pigments of Comparative Examples 3 to 8 and showed adequate thermal insulation properties. Further, as to the thermal insulation paint compositions of Examples 14, 15, 17 and 18 that respectively contained the bright pigments of Examples 7, 9, 10 and 12 having an average inorganic base thickness of 0.2 $\mu$m or more and less than 1.0 $\mu$m, they

had a higher near-infrared reflectivity and higher thermal insulation properties, as shown in Table 5.

Industrial Applicability

Since the bright pigment of the present invention exhibits a high luminance design, it can be preferably used as an ingredient of, for example, cosmetic, paint, resin and ink compositions, artificial marble molded products and coated paper. Further, the bright pigment of the present invention can also be preferably used as an ingredient of a thermal insulation paint composition or an identification ink composition because of its effect of reflecting near-infrared radiation.

Description of Reference Numerals

1     bright pigment

10    inorganic base

20    silver-containing coating

30    luminance meter

40    light source

50    coating film

**Claims**

**1.**  A bright pigment comprising:

a scaly inorganic base; and
a coating containing a pure substance of silver or a silver alloy and covering the inorganic base,
the inorganic base having an average thickness of 0.2 $\mu$m to 7.0 $\mu$m, wherein the content of the pure substance of silver or the silver alloy in the bright pigment satisfies the formulas 1) and 2):

1)

$$y \geq -9 \operatorname{Ln}(x) + 19$$

2)

$$y \leq -22 \operatorname{Ln}(x) + 52$$

where x is the average thickness ($\mu$m) of the inorganic base and y is the content (mass%) of the pure substance of silver or the silver alloy

**2.**  The bright pigment according to claim 1, wherein the inorganic base has an average thickness of 0.2 $\mu$m or more and less than 1.0 $\mu$m.

**3.**  The bright pigment according to claim 1 or 2, wherein the inorganic base has an average particle size of 10 $\mu$m to 200 $\mu$m.

**4.**  The bright pigment according to any one of claims 1 to 3, wherein the inorganic base includes at least one selected from the group consisting of glass, natural mica, synthetic mica, silica and alumina.

5. A cosmetic composition comprising the bright pigment according to any one of claims 1 to 4.

6. The cosmetic composition according to claim 5, further comprising an organic colorant.

7. A paint composition comprising the bright pigment according to any one of claims 1 to 4.

8. The paint composition according to claim 7, further comprising an organic colorant.

9. A thermal insulation paint composition comprising the bright pigment according to any one of claims 1 to 4.

10. The thermal insulation paint composition according to claim 9, further comprising an organic colorant.

11. An ink composition comprising the bright pigment according to any one of claims 1 to 4.

12. The ink composition according to claim 11, further comprising an organic colorant.

13. A resin composition comprising the bright pigment according to any one of claims 1 to 4.

14. The resin composition according to claim 13, further comprising an organic colorant.

1

20

a

10

FIG. 1

30

40

5°

50

FIG. 2

FIG. 3

<div style="text-align:center">

**INTERNATIONAL SEARCH REPORT**

</div>

| International application No. |
| --- |
| PCT/JP2009/064802 |

**A. CLASSIFICATION OF SUBJECT MATTER**
*C09C3/06*(2006.01)i, *A61K8/19*(2006.01)i, *A61K8/25*(2006.01)i, *A61K8/26*
(2006.01)i, *A61Q1/02*(2006.01)i, *A61Q3/02*(2006.01)i, *C09C1/00*(2006.01)i,
*C09D5/29*(2006.01)i, *C09D5/33*(2006.01)i, *C09D7/12*(2006.01)i,
According to International Patent Classification (IPC) or to both national classification and IPC

**B. FIELDS SEARCHED**

Minimum documentation searched (classification system followed by classification symbols)
C09C3/06, A61K8/19, A61K8/25, A61K8/26, A61Q1/02, A61Q3/02, C09C1/00,
C09D5/29, C09D5/33, C09D7/12, C09D11/00, C09D201/00

Documentation searched other than minimum documentation to the extent that such documents are included in the fields searched
Jitsuyo Shinan Koho          1922-1996   Jitsuyo Shinan Toroku Koho   1996-2009
Kokai Jitsuyo Shinan Koho    1971-2009   Toroku Jitsuyo Shinan Koho   1994-2009

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| X<br>Y | WO 2007/054379 A1  (CIBA SPECIALTY CHEMICALS<br>HOLDING INC.),<br>18 May 2007 (18.05.2007),<br>claims 1, 11; P5 L1-L13, P16 L2-6<br>& JP 2008-546880 A     & EP 1904586 A<br>& KR 10-2008-0017485 A   & CN 101203574 A | 1-14<br>7-14 |
| X<br>Y | JP 2002-138010 A (Nippon Sheet Glass Co.,<br>Ltd.),<br>14 May 2002 (14.05.2002),<br>claims 1 to 3; examples<br>(Family: none) | 1-6<br>7-14 |

| ☒ | Further documents are listed in the continuation of Box C. | ☐ | See patent family annex. |
| --- | --- | --- | --- |

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>    24 November, 2009 (24.11.09) | Date of mailing of the international search report<br>    08 December, 2009 (08.12.09) |
| --- | --- |
| Name and mailing address of the ISA/<br>    Japanese Patent Office | Authorized officer |
| Facsimile No. | Telephone No. |

Form PCT/ISA/210 (second sheet) (April 2007)

**INTERNATIONAL SEARCH REPORT**

International application No.

PCT/JP2009/064802

C (Continuation).　DOCUMENTS CONSIDERED TO BE RELEVANT

| Category* | Citation of document, with indication, where appropriate, of the relevant passages | Relevant to claim No. |
|---|---|---|
| X<br>Y | JP 2001-089324 A　(L'Oreal),<br>03 April 2001 (03.04.2001),<br>claims 1 to 20; examples<br>& JP 2005-225893 A　　　& US 6491932 B1<br>& EP 1082952 A1　　　& DE 60000253 D<br>& DE 60000253 T　　　& FR 2798062 A<br>& FR 2798062 A1　　　& BR 4110 A<br>& AT 220315 T　　　& ES 2179806 T<br>& CN 1286974 A | 1-6<br>7-14 |

Form PCT/ISA/210 (continuation of second sheet) (April 2007)

**INTERNATIONAL SEARCH REPORT**

International application No.

PCT/JP2009/064802

Continuation of A. CLASSIFICATION OF SUBJECT MATTER
  (International Patent Classification (IPC))

C09D11/00(2006.01)i, C09D201/00(2006.01)i

(According to International Patent Classification (IPC) or to both national classification and IPC)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H9188830 A
- JP 2001089324 A
- JP 2002138010 A
- JP H10158540 A
- JP H676292 B